## Europäisches Patentamt

## European Patent Office

## Office européen des brevets

(19)

(11) Publication number: **0 227 619**
**A2**

(12)

# EUROPEAN PATENT APPLICATION

(21) Application number: **86850417.6**

(22) Date of filing: **02.12.86**

(51) Int. Cl.⁴: **C 07 K 7/10**
**A 61 K 37/36, G 01 N 33/53**

(30) Priority: **13.12.85 SE 8505920**

(43) Date of publication of application:
**01.07.87 Bulletin 87/27**

(84) Designated Contracting States:
**AT BE CH DE ES FR GB GR IT LI LU NL SE**

(71) Applicant: **KabiGen AB**
**S-112 87 Stockholm (SE)**

(72) Inventor: **Sara, Vicki Rubian**
**Rörstrandsgatan 32**
**S-113 40 Stockholm (SE)**

(74) Representative: **Burman, Tore et al**
**Bergling & Sundbergh AB P.O. Box 7645**
**S-103 94 Stockholm (SE)**

(54) **New protein and its use.**

(57) Protein comprising at the N-terminal thereof the amino acid sequence:
NH$_2$-Thr$^1$-Leu-Cys-Gly-Ala-Glu-Leu-Val-Asp-Ala$^{10}$-Leu-Gln-Phe-Val-Cys-Gly-Asp-Arg-Gly-Phe$^{20}$-Tyr-Phe-Asn-Lys-Pro-Thr-Gly-Tyr-Gly-R wherein R is an amino acid sequence such that the overall apparent molecular weight of the protein is about 7000:
method of stimulating brain cell growth;
composition for therapeutic or diagnostic use; and
a method for diagnosing the presence of brain cell growth factor.

EP 0 227 619 A2

**Description**

New protein and its use.

The present invention relates to a new and novel protein and its therapeutic and diagnostic use.

The somatomedins are a family of growth-promoting peptide hormones (Hall, K. and Sara, V.R. in: Aurbach, G.D. (Ed.) Vitamins and hormones. 40:175, 1983). In adult human plasma, two somatomedins, namely insulin-like growth factor 1 and 2 (IGF-1 and IGF-2) have been isolated and characterized (Rinderknecht, E. and Humbel, R.E. J.Biol.Chem., 253:2769, 1978a.; Rinderknecht E. and Humbel, R.E. FEBS Lett. 89:283, 1978b.). Somatomedins A (SMA) and C (SMC) are identical to IGF-1 (Enberg, G., Carlquist, M., Jörnvall, H. and Hall, K. Eur.J.Biochem., 143:117, 1984; Svoboda, M.E., Van Wyk, J.J., Klapper, D.G., Fellows, R.E., Grissom, F.E. and Schlueter, R.J. Biochemistry, 19:790, 1980). An additional fetal form of somatomedin has been proposed to exist in man (Sara, V.R. and Hall, K. Clin.Obst.& Gynecol. 23:765, 1980; Sara, V.R., Hall, K., Rodeck, C.H. and Wetterberg, L. ProcNatl.Acad.Sci., USA, 78:3175, 1981). This hypothesis was based upon failure to detect either immunoreactive IGF-1 or IGF-2 in human fetal serum (Sara, V.R., Hall,K., Rodeck,C.H. and Wetterberg,L. Proc.Natl.Acad.Sci.,USA 78:3175, 1981; Sara,V.R. and Hall,K. in:Fetal Neuroendocrinology. P.Gluckman and F.Ellendorf (Eds). Perinatal Press, N.Y.(in press)) in spite of the highest concentration of somatomedin receptors being found in fetal tissues (Sara, V.R., Hall,K., Mizaki,M., Fryklund, L., Christensen,N. and Wetterberg, L. J.Clin.Invest. 71:1084, 1983) as well as a direct growth-promoting action being demonstrated in vitro (Sara, V.R., Hall,K., Ottosson-Seeberger,A. and Wetterberg,L. in:Cumming, I.A., Funder,J.W., Mendelsohn,F.A.O. (Eds.) Endocrinology 1980. Canberra:A.A.S. pp.453, 1980). Additionally, the somatomedin receptor in human fetal membranes early in gestation showed characteristics incompatible with its classification as either a type 1 or 2 somatomedin receptor.

The present invention has for its principle object to provide a new somatomedin related to IGF-1 but being constituted by a human fetal somatomedin.

Another object of the invention is to provide a new somatomedin having the capacity of stimulating nervous system growth such as brain cell growth in mammals including man.

Having these and other objects in mind a new somatomedin has now been found which at the N-terminal thereof has the amino acid sequence:

$NH_2$-Thr[1]-Leu-Cys-Gly-Ala-Glu-Leu-Val-Asp-Ala[10]-Leu-Gln-Phe-Val-Cys-Gly-Asp-Arg-Gly-Phe[20]-Tyr-Phe-Asn-Lys-Pro-Thr-Gly-Tyr-Gly-R.

In this protein R is an amino acid sequence such that the overall apparent molecular weight of the protein is about 7000.

R in the protein of the invention is preferably such that the overall composition of the protein corresponds to that of IGF-1 except for the three N-terminal amino acid residues of IGF-1 which are not found in the protein of this invention.

R is particularly such that the overall structure of the protein corresponds to that of IGF-1 excluding the three N-terminal amino acid residues of IGF-1. Thus, in its preferred form, the protein of this invention has the following amino acid sequence and composition:

$NH_2$-Thr[1]-Leu-Cys-Gly-Ala-Glu-Leu-Val-Asp-Ala[10]-Leu-Gln-Phe-Val-Cys-Gly-Asp-Arg-Gly-Phe[20]-Tyr-Phe-Asn-Lys-Pro-Thr-Gly-Tyr-Gly-Ser-Ser-Ser-Arg-Arg-Ala-Pro-Gln-Thr-Gly-Ile-Val-Asp-Glu-Cys-Cys-Phe-Arg-Ser-Cys-Asp-Leu-Arg-Arg-Leu-Glu-Met-Tyr -Cys-Ala-Pro-Leu-Lys-Pro-Ala-Lys-Ser-Ala.

The present invention also includes a method of stimulating neural tissue growth, such as brain cell growth in mammals including man, and such method involves the administration of an effective amount of the protein of the invention to a mammal subject to treatment.

According to another aspect of the invention there is provided a composition for therapeutic or diagnostic use comprising in combination an active amount of the protein of the invention in combination with a therapeutically or diagnostically acceptable carrier therefor. By the term "therapeutically and diagnostically acceptable" is meant that the carrier is non-toxic and non-interfering in the applications where the composition of the invention is useful.

The new protein of the present invention may thus in accordance with traditional pharmaceutical practice be formulated for use in human or veterinary medicine for therapeutic or diagnostic purposes. Such compositions may include the active protein in combination with a pharmaceutically or diagnostically acceptable carrier, which may be solid, semisolid or liquid, and in some cases the protein according to the invention can be used as such without dilution.

The compositions of the invention include those in a form adapted for oral or parenteral use.

Suitable forms of the composition of this invention include tablets, capsules, sirups, suspensions, solutions, and forms suitable for injection or infusion. Such compositions may contain conventional pharmaceutically acceptable materials, such as diluents, binders, colours, flavours, preservatives, disintegrants and the like in accordance with conventional pharmaceutical practice in the manner well understood by those skilled in the art of formulating drugs.

Injectable or infusable compositions of a structural element of the invention are particularly suitable as useful blood-levels of the protein of the invention can occur after administration by injection or infusion.

According to still another aspect of the invention there is provided a method for diagnosing the presence of brain cell growth factor in physiological fluids and tissues using the immunological interaction between antibodies generated by the protein of the invention and antigen comprised in a fluid or tissue sample subject to diagnosis. Such method can be designed as a procedure wherein antibodies to the protein of the invention are generated and the inhibition of interaction between such antibodies and a label protein of the invention is recorded by adding therein amounts of a fluid or tissue sample subject to test. This method thus enables quantitative or at least semi-quantitative determination of brain cell growth factor in samples of interest.

The invention will in the following be described by non-limiting examples with reference to the appended drawings, wherein:

Fig. 1 is a diagram showing separation by a column chromatography of the protein of the invention;

Fig. 2 shows a diagram on separation of eluted material on another chromatographic column;

Fig. 3 is a diagram on reverse phase chromatography of protein of the invention;

Fig. 4 is a diagram showing crossreactions of the protein of the invention and IGF-1 and IGF-2;

Fig. 5 is a diagram showing the biological action of peptide on fetal brain cell DNA synthesis;

Fig. 6 shows the amino acid sequence of the peptide of the invention compared to that of IGF-1; and

Fig. 7 is a corresponding comparison but involving IGF-2.

## EXAMPLE 1

### Preparation of cytosol from human fetal brain tissue

Immediately after legal prostaglandin abortion, fetuses were measured and stored in sterile saline at $+4°C$. Within 12 hours after abortion, the whole fetal brain minus cerebellum was removed and stored at $-70°C$ until use. Five volumes of 50 mM Tris-HCl buffer containing 0.2 mM PMSF (phenylmethylsulfonylfluorid) was added to frozen brain tissue which was then homogenized with a Polytron for 60 seconds. The homogenate was centrifuged at 50,000g for 15 minutes and the supernatant collected and stored at $-85°C$ until use.

## EXAMPLE 2

### Purification

The purification procedure from human fetal brain cytosol consisted of the following steps. Activity was monitored by fetal brain RRA-IGF-1 and protein according to Kalb, v.F. & Bernhohr, R.W. Anal.Biochem 82, 362-371, 1977.

All glassware was siliconized and procedures were carried out at $+4°C$. All reagents were of analytical grade.

## EXAMPLE 3

### Acid Precipitation and Sephadex G-50 Chromatography.

0.2 mM PMSF and 0.1 µM Pepstatin were added to cytosol during thawing. Cytosol was precipitated by lowering the pH to 5.5 with 1M acetic acid. After centrifugation at 16,000 g for 30 minutes, the supernatant was further acidified to 0.1 M acetic acid and stirred overnight at $+4°C$. After centrifugation at 16,000 g for 30 minutes the supernatant was concentrated approximately 3 times, dialyzed overnight against 3x10 volumes of 0.1 M acetic acid, further concentrated and then chromatographed on a column (3x150 cm) of Sephadex G-50 fine (Pharmacia, Sweden) equilibrated in 0.1 M acetic acid. Markers for calibration were blue dextran and $CoCl_2$. The column was operated at a flow rate of 24 ml/h.

## EXAMPLE 4

Amniotic fluid somatomedin carrier protein coupled to CNBr-activated Sepharose 4B (Pharmacia, Sweden) was obtained from Prof. Kerstin Hall and Dr. Guilherme Póvoa, Karolinska Hospital, Sweden. Pooled active fractions from the Sephadex G-50 column were concentrated, dialyzed overnight against 5 volumes of 50 mM Tris-HCL pH 7.7 and applied to the affinity column (6x20 mm) equilibrated in 50 mM Tris-HCL at pH 7.7. The column was washed with 3 ml of Tris-buffer and 3 ml $H_2O$. Active material was eluted with 6 ml 1M acetic acid and the eluate was frozen.

## EXAMPLE 5

### Cation exchange FPLC

Active fractions from the affinity chromatography step were pooled, concentrated and applied to a fast protein liquid chromatography system using an HR 5/5 column of Mono S (Pharmacia, Sweden) equilibrated with 10 mM ammonium acetate pH 6.5 and 10% acetonitrile. Elution was performed at a flow rate of 1.0 ml/min with a linear gradient of ammonium acetate at pH 6.5 in 10% acetonitrile.

## EXAMPLE 6

### Reverse phase HPLC

Active lyophilized material was resuspended in 30% acetic acid and applied to an Ultropac TSK ODS 120T column (4.6x200 mm) particle size 5 µm) (LKB, Bromma, Sweden). The column was eluted with a linear gradient of acetonitrile in 0.1% trifluoroacetic acid at a flow rate of 1.0 ml/min.

## EXAMPLE 7

### Amino acid composition

Samples of active lyophilized material fromExample 6 above were hydrolyzed for 24 h at 110°C with 6 M HCl/0.5% phenol in evacuated tubes. Liberated amino acids were converted to phenylthiocarbamyl derivatives by coupling with phenylisothiocyanate, and phenylthiocarbamyl derivatives were identified by reverse phase high-performance liquid chromatography (Waters) on a $C_{18}$ column (Spherisorb S3 ODS 2; 3 μm; Phase Separations, Queensferry, UK) using an acetonitrile gradient in a sodium phosphate buffer.

## EXAMPLE 8

Samples for sequence analysis were degraded in an Applied Biosystems 470A gas-phase sequencer.

## EXAMPLE 9

### Fetal Brain Radioreceptorassay (fetal brain RRA-IGF1).

This assay is described in detail in Sara, Hall, Rodeck and Wetterberg (Sara, V.R., Hall, K., Rodeck, C.H. and Wetterberg, L. Proc.Natl.Acad.Sci., USA, 78:3175, 1981). Human fetal brain plasma membrane is used as matrix. Purified IGF-1 was iodinated by the lactoperoxidase method and used as ligand. Purified IGF-1 and IGF-2 prepared according to methods detailed earlier (Enberg, G., Carlquist, M., Jörnvall, H. and Hall, K. Eur.J.Biochem., 143:117, 1984) were obtained from Dr. Gösta Enberg, Karolinska Hospital, Sweden. Values are expressed in relation to a human reference serum standard which is given an arbitrary value of 1U/ml.

## EXAMPLE 10

### Bioassay for Growth-Promoting Activity

Biological activity was assessed by determining the incorporation of $^3$H-thymidine into fetal rat brain cell DNA in an in vitro system according to Sara, V.R., King, T.L. Stuart, M.C. & Lazarus, L., Endocrinology 99: 90-97, 1976. A primary cell suspension is prepared from fetal rat brains on day 18 of gestation. After incubation in a serum-free medium containing $^3$H-thymidine for 20 hours at +37°C, DNA is extracted from the cells and the incorporation of $^3$H-thymidine into DNA determined.

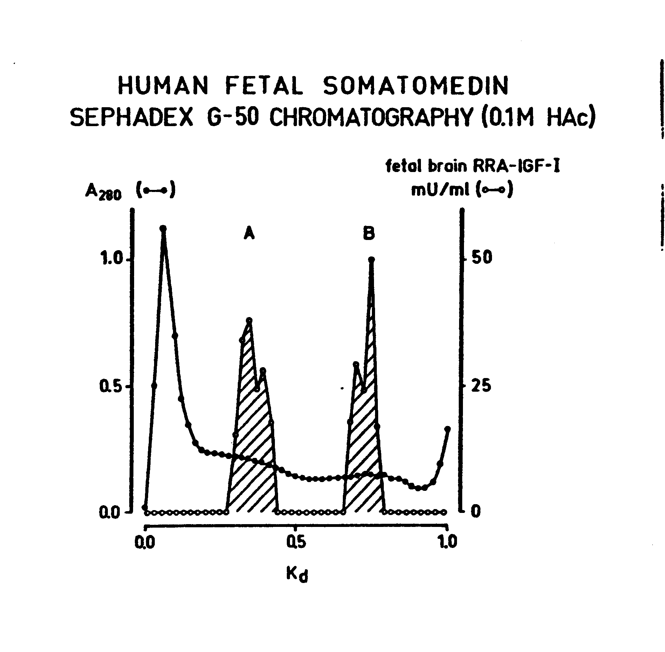

The purification and yield of fetal brain radioreceptorassayable activity from human fetal brain cytosol is given in appended Table 1. After acid precipitation to separate somatomedins from their binding proteins, cytosol was subjected to gel chromatography on Sephadex G-50. The column was eluted with 0.1 M acetic acid. A representative chromatogram is illustrated in Fig. 1. Fig. 1 illustrates separation on a column of Sephadex G-50 (3x150 cm) equilibrated with 0.1 M acetic acid. Fetal brain RRA-IGF-1 activity eluting as pools A and B is shown by the hatched area. Fetal brain RRA-IGF-1 activity eluted as two peaks with apparent molecular weights of 12K and 4K, respectively. These were termed Pool A and B and crossreacted only slightly or not at all with IGF-1 or IGF-2 polyclonal antibodies.

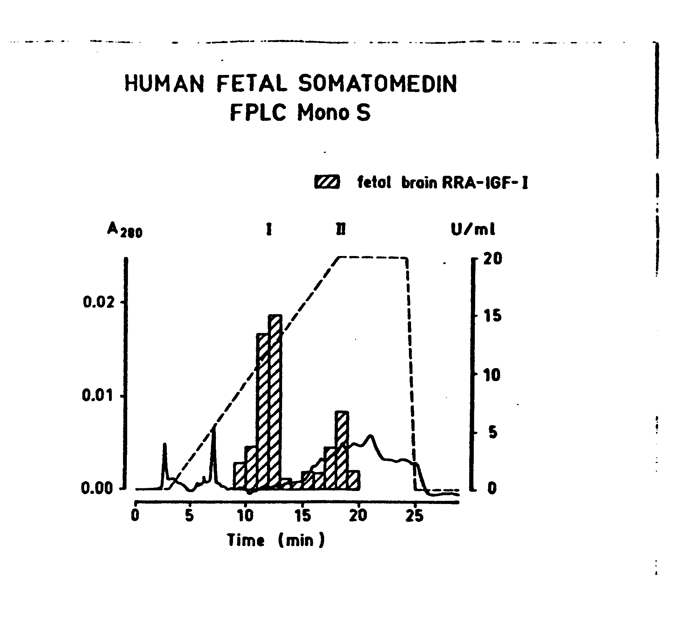

B Pool activity was further purified by affinity chromatography. Activity was eluted from this column with 1M acetic acid. This eluate was then concentrated and chromatographed on FPLC Mono S as illustrated in Fig. 2. This procedure consisted in separation of the active material eluted from the affinity chromatography column on an HR 5/5 column of Mono S. Elution was performed with a linear gradient of 10-300 mM ammonium acetate in 10% acetonitrile at pH 6.5 (dotted line). Fetal brain RRA-IGF-1 activity eluting as Peaks I and II is shown by the hatched area. It can be seen from Fig. 2 that two peaks of fetal brain RRA-IGF-1 activity eluted from the column. Peaks I and II were pooled, lyophilized and further purified by HPLC reverse phase using an Ultropac $C_{18}$ column.

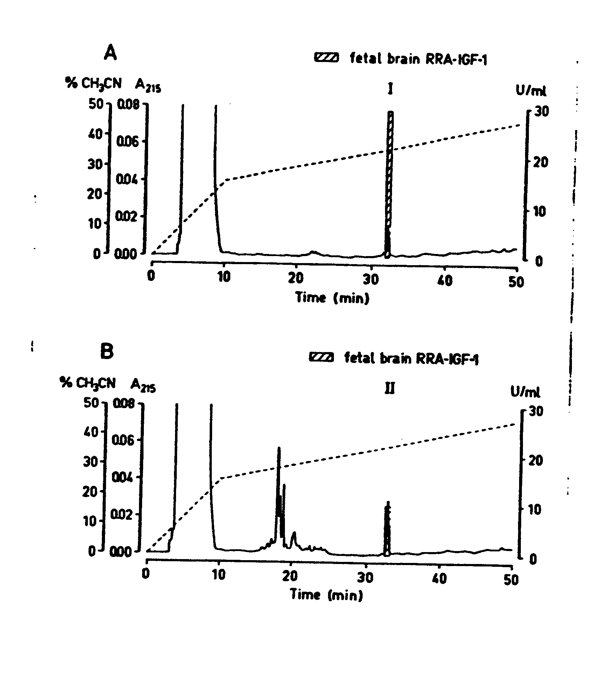

The elution pattern for both peaks is given in Fig. 3 illustrating reverse phase HPLC (Ultropac TSK ODS 120T) chromatography of Peaks I (A,upper part) and II (B, lower part) derived from cation exchange FPLC. Elution was performed with a gradient of 0-45% acetonitrile in 0.1% trifluoroacetic acid (dotted line). Fetal brain RRA-IGF-1 activity is shown by the hatched area. The figure shows a single peak of fetal brain RRA-IGF-1 activity which eluted at approximately 37% acetonitrile with Peak I eluting slightly before Peak II.

The specific activities of peptide I and II were approximately 50,000 U/mg protein and 5,000 U/mg protein respectively. The protein content was estimated by acid hydrolysis. The purity of these peptides was confirmed by $NH_2$-terminal analyses. This purification has been reproduced in three separate preparations.

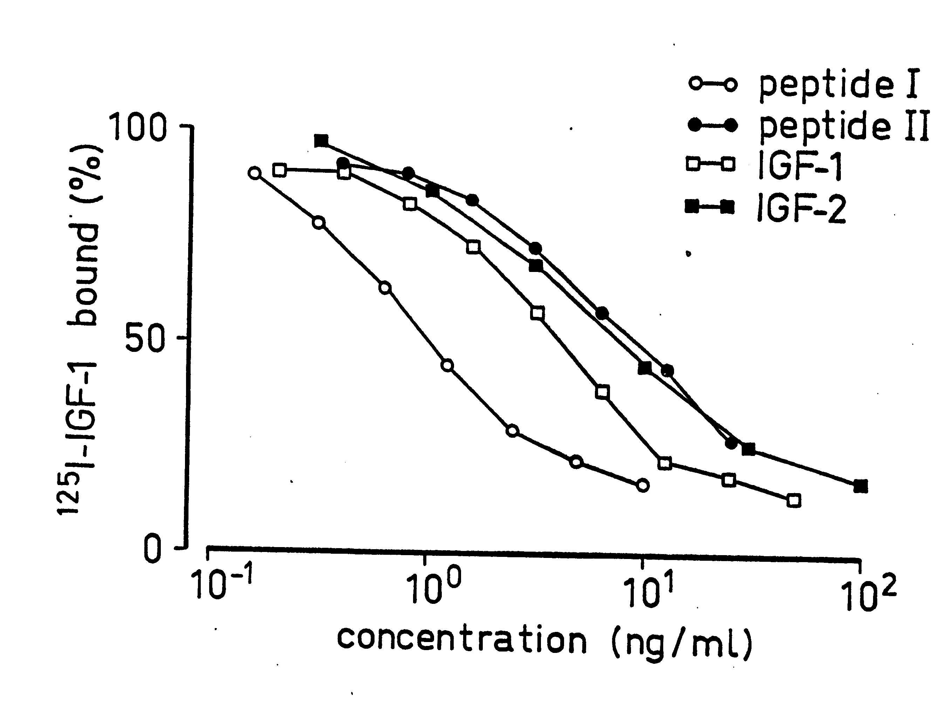

The crossreaction of Peptide I and II as well as IGF-1 and IGF-2 purified from serum in the fetal brain RRA-IGF-1 is given in Fig 4. The test is based on displacement of $^{125}$-IGF-1 from human fetal brain plasma membranes (330 μg protein/ml) by Peptides I (o— —o) and II (● — —●) compared to IGF-1 (□ — —□) and IGF-2 (■ — —■). In this assay, purified IGF-1 and IGF-2 purified from serum have an activity of 10,000 U/mg and 5,000 U/mg respectively. Thus in this assay, peptide I is approximately 5 and 10 times more potent in crossreaction than IGF-1 and IGF-2 respectively and peptide II is equipotent to IGF-2.

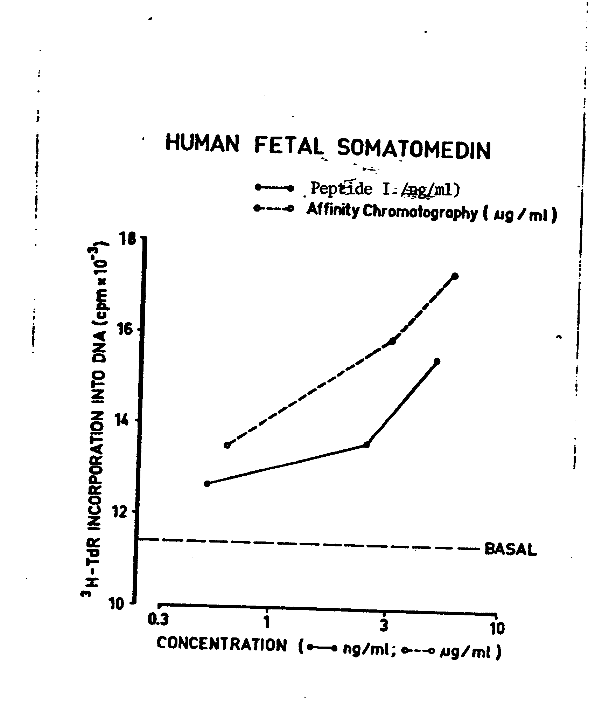

The biological activity of peptide I was determined. The effect on $^3$H-thymidine incorporation into fetal rat brain DNA is shown in Fig. 5. Varying concentrations of the eluate after binding protein affinity chromatography (o— —o, μg/ml) and Peptide I after HPLC reverse phase (● — — ●, ng/ml) were incubated for 20 hrs at 37°C

4

with fetal rat brain cells and the incorporation of $^3$H-TdR into extracted DNA was determined. The finally purified material had a potent stimulatory effect on $^3$H-thymidine uptake into DNA, being active between 1-3 ng/ml. This is tenfold more potent than purified IGF-1 or IGF-2 which are equipotent in this bioassay having a significant effect between 10-30 ng/ml. Biological activity after affinity chromatography was also examined. As shown in Fig. 5, this material also significantly stimulated DNA synthesis but was several hundredfold less potent. This potency difference corresponds to the approximate degree of purification between these stages (Table 1).

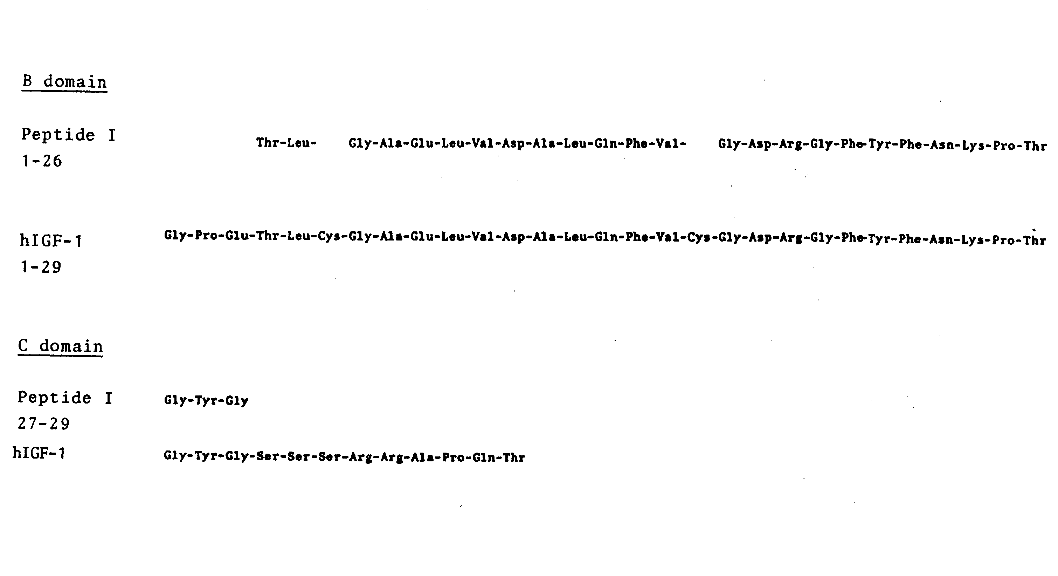

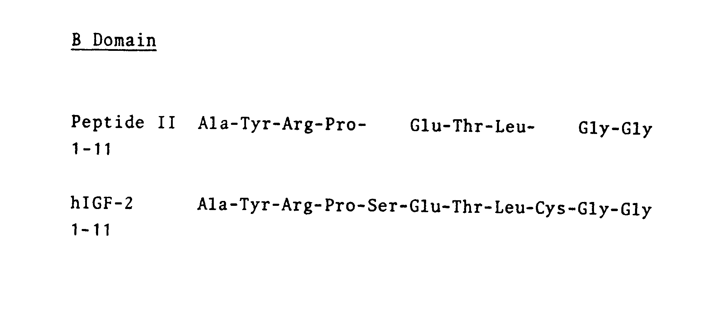

The amino acid composition of Peptide I and II as well as IGF-1 and IGF-2 is given in Table 2. Within the experimental error these results suggest that Peptide I and II have almost identical amino acid compositions to IGF-1 and IGF-2 respectively. However structural analysis of the amino acid sequences revealed N-terminal differences between Peptide I and IGF-1 (Fig. 6 Amino acid sequence of Peptide I compared to the amino acid sequence of IGF-1). The N-terminal threonine of Peptide I aligned with position 4 in IGF-1. With this alignment, identical amino acid residues were observed, so that positions 1 to 29 in Peptide I were identical to positions 4-32 of IGF-1. N-terminal analysis revealed homogeneity of the preparations. As shown in Fig. 7, residues 1 to 11 of Peptide II were identical to those of IGF-2.

The protein of the present invention which has been found in human fetal brain tissue and has been identified according to the present disclosure is more potent in crossreaction in the fetal brain radioreceptor assay and has been shown to possess a potent biological action on fetal brain cell proliferation in vitro. These findings indicate utility of the protein for therapeutic and diagnostic applications, i.e. stimulating nervous system growth and diagnosis for the presence of growth factor in physiological fluids and tissues, respectively.

In regard to the diagnosis aspect to the invention immunological technique for the measurement of presence of brain cell growth factor in physiological fluids and tissues can be used. For quantitative purposes immunoassay technique can be applied based on the ability of the brain somatomedin variant to inhibit the formation of a complex of labelled protein and specific antibody. In such system the concentration of the antigen in an unknown sample is determined by comparison with a standard response curve established by measurement of either the bound antibody or the free form.

While the invention has been described with reference to specific measures, starting materials etc. it is to be noted that the invention is not limited otherwise than as defined in the appended claims.

Table 1.   The purification and yield of fetal brain
           RRA-IGF-1 from human fetal brain cytosol.

| Purification Step | Specific Activity (U/mg) | Purification | Recovery (%) |
|---|---|---|---|
| Cytosol | 0.04 | 1.0 | 100 |
| Acid precipitation | 0.07 | 1.8 | 60 |
| G-50   A pool | 0.20 | 5.0 | 30 |
|        B pool | 0.50 | 12.5 | 12 |
|        B pool | | | |
| Affinity chromatography | 56 | 1,400 | 12 |
| FPLC   Mono S | - a) | - a) | 11 |
| HPLC   Ultropac   I | 50,000 | 1,250,000 | 5 |
|               II | 5,000 | 125,000 | 5 |

a) Protein content not determined.

**0 227 619**

Table 2. Total compositions of Peptide I and II as obtained by acid hydrolysis, compared to those of IGF-1 and IGF-2 obtained from sequence analysis.

| Residue | Peptide I | Peptide II | IGF-1 | IGF-2 |
|---------|-----------|------------|-------|-------|
| Asx | 3.9 | 3.1 | 5 | 3 |
| Glx | 6.0 | 5.5 | 6 | 7 |
| Ser | 6.0 | 4.6 | 5 | 7 |
| Gly | 6.1 | 3.8 | 7 | 5 |
| Thr | 2.8 | 3.8 | 3 | 4 |
| Ala | 5.3 | 4.7 | 6 | 5 |
| His | 0.0 | 0.0 | 0 | 0 |
| Pro | 3.3 | 2.9 | 5 | 3 |
| Arg | 6.3 | 8.6 | 6 | 8 |
| Tyr | 1.7 | 3.4 | 3 | 3 |
| Val | 3.1 | 4.5 | 3 | 4 |
| Met | 0.1 | 0.1 | 1 | 0 |
| Cys | n.d. | n.d. | 6 | 6 |
| Isoleu | 0.9 | 0.9 | 1 | 1 |
| Leu | 5.6 | 7.1 | 6 | 6 |
| Phe | 3.5 | 4.0 | 4 | 4 |
| Lys | 1.9 | 1.1 | 3 | 1 |

**Claims**

1. Protein comprising at the N-terminal thereof the amino acid sequence:
$NH_2$-$Thr^1$-Leu-Cys-Gly-Ala-Glu-Leu-Val-Asp-$Ala^{10}$-Leu-Gln-Phe-Val-Cys-Gly-Asp-Arg-Gly-$Phe^{20}$-Tyr-Phe-Asn-Lys-Pro-Thr-Gly-Tyr-Gly-R wherein R is an amino acid sequence such that the overall apparent molecular weight of the protein is about 7000.

2. Protein according to claim 1, wherein R is such that the overall composition of the protein corresponds to that of IGF-1 excluding the three N-terminal amino acid residues thereof.

3. Protein according to claim 2, wherein R is such that the overall structure of the protein corresponds to that of IGF-1 excluding the three N-terminal amino acid residues thereof.

4. A method of stimulating neural tissue growth in mammals including man, comprising administering an effective amount of the protein of claim 1, 2 or 3 to a mammal subject to treatment.

5. A composition for therapeutic or diagnostic use comprising in combination an active amount of the protein of any of claims 1 to 3 in combination with a nontoxic and noninterfering carrier therefor.

7

6. A method for diagnosing the presence of neural tissue growth factor in physiological fluids and tissues using the immunological interaction between antibodies generated by the protein of claim 1 and antigen comprised in a fluid or tissue sample subject to diagnosis.

7. A method according to claim 6 comprising the steps:

a) generating antibodies to the protein of claim 1;

b) recording inhibition of interaction between antibodies obtained under step a) and a labelled protein according to claim 1 by adding varying amounts of a fluid or tissue sample subject to test.

8. A method according to claim 4 applied to brain cell growth.

9. A method according to claim 6 applied to brain cell growth.

HUMAN FETAL SOMATOMEDIN
SEPHADEX G-50 CHROMATOGRAPHY (0.1M HAc)

**HUMAN FETAL SOMATOMEDIN
FPLC Mono S**

0227619

A

% CH₃CN  A₂₁₅          ▨ fetal brain RRA-IGF-1

                              I                        U/ml

Time (min)

B

% CH₃CN  A₂₁₅          ▨ fetal brain RRA-IGF-1

                              II                       U/ml

Time (min)

peptide I

peptide II

IGF-1

IGF-2

$^{125}I$-IGF-1 bound (%)

concentration (ng/mL)

0227619

# HUMAN FETAL SOMATOMEDIN

●——● Peptide I. (ng/ml)
●--● Affinity Chromatography ( μg / ml )

BASAL

$^{3}$H-TdR INCORPORATION INTO DNA (cpm × $10^{-3}$)

CONCENTRATION ( ●——● ng/ml; ●--● μg/ml )

### B domain

Peptide I
1-26

Thr-Leu- Gly-Ala-Glu-Leu-Val-Asp-Ala-Leu-Gln-Phe-Val- Gly-Asp-Arg-Gly-Phe-Tyr-Phe-Asn-Lys-Pro-Thr

hIGF-1
1-29

Gly-Pro-Glu-Thr-Leu-Cys-Gly-Ala-Glu-Leu-Val-Asp-Ala-Leu-Gln-Phe-Val-Cys-Gly-Asp-Arg-Gly-Phe-Tyr-Phe-Asn-Lys-Pro-Thr

### C domain

Peptide I
27-29

Gly-Tyr-Gly

hIGF-1

Gly-Tyr-Gly-Ser-Ser-Ser-Arg-Arg-Ala-Pro-Gln-Thr

## B Domain

| Peptide II 1-11 | Ala-Tyr-Arg-Pro- | Glu-Thr-Leu- | Gly-Gly |

hIGF-2
1-11      Ala-Tyr-Arg-Pro-Ser-Glu-Thr-Leu-Cys-Gly-Gly